# EUROPÄISCHE PATENTSCHRIFT

(11) **EP 0 433 846 B1**
(45) Veröffentlichungstag und Bekanntmachung des Hinweises auf die Patenterteilung: **18.03.1998**
(21) Anmeldenummer: 90123794.1
(22) Anmeldetag: 11.12.1990
(51) Int. Cl.: C07D 473/40

(54) **Verfahren zur Herstellung von 2-Acylamino-6-halogen-purin aus 2,9-Diacylguanin**
Process for the preparation of 2-acylamino-6-halogeno-purine from 2,9-diacylguanine
Procédé pour la préparation de la 2-acylamino-6-halogéno-purine à partir de la 2,9-diacylguanine

(30) Priorität: 16.12.1989 DE 3941657
(43) Veröffentlichungstag der Anmeldung: 26.06.1991
(73) Patentinhaber: HOECHST AKTIENGESELLSCHAFT, 65926 Frankfurt am Main (DE)
(72) Erfinder: Hertzsch, Winfried, Dr., W-6000 Frankfurt am Main (DE)

(56) Entgegenhaltungen:
- EP-A- 0 203 685
- US-A- 4 405 781

## Beschreibung

2-Acylamino-6-halogen-purine sind für die pharmazeutische Industrie im Bereich der Nucleosidsynthesen sehr vielseitig einsetzbare Zwischenprodukte.
2-Acetamino-6-chlor-purin ist eine in der Literatur bekannte Verbindung. Als Ausgangsmaterial für seine Synthese dient z.B. 2-Amino-6-oxo-purin (Guanin), das über Chlorierung zu 2-Amino-6-chlor-purin mit anschließender Acetylierung in 2-Acetamino-6-chlor-purin umgewandelt wird. In der Europäischen Patentanmeldung 0 203 685 wird ein Verfahren zur Herstellung von 2-Amino-6-chlor-purin aus Guanin beschrieben. Dazu wird Guanin in Acetonitril zusammen mit Phosphoroxychlorid und Tetraethylammoniumchlorid bei Rückflußtemperatur chloriert. Nach 24 Stunden kontinuierlicher Extraktion erhält man 2-Amino-6-chlor-purin mit einer Ausbeute von 42 %. Die Verwendung von Acetanhydrid in N,N-Dimethylacetamid zur Acetylierung von 2-Amino-6-chlor-purin zu 2-Acetamino-6-chlor-purin wird von W.A. Bowles et al. beschrieben (J. Med. Chem. 6, 1963, 471-480). Dabei wird bei 150°C eine Ausbeute von 44 % erreicht. Der Syntheseweg von 2-Acetamino-6-chlor-purin, der von Guanin ausgeht und über 2-Amino-6-chlor-purin führt, ist mit hohen Ausbeuteverlusten verbunden und durch lange Extraktionszeiten charakterisiert.
Überraschend wurde nun gefunden, daß durch Halogenierung von 2,9-Diacylguanin (2-Acylamino-6-oxo-9-acyl-purin) in hohen Ausbeuten 2-Acylamino-6-halogen-purin hergestellt werden kann. Die Erfindung betrifft somit ein Verfahren zur Herstellung der Verbindung der Formel I, 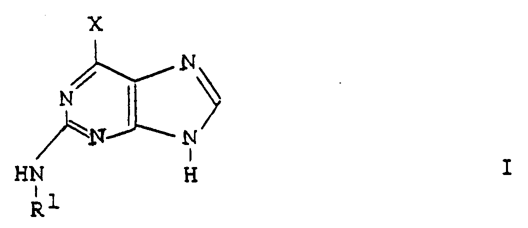 in der R¹ für eine geradkettige oder verzweigte Alkanoylgruppe mit 2 bis 10 Kohlenstoffatomen oder eine Aralkanoylgruppe mit 7 bis 10 Kohlenstoffatomen und X für Chlor oder Brom steht, das dadurch gekennzeichnet ist, daß 2,9-Diacylguanin der Formel II, 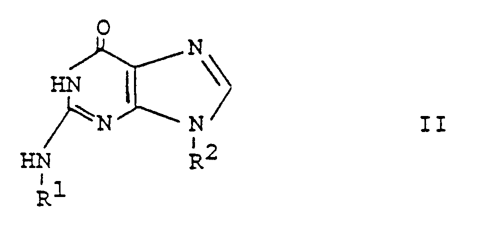 in der R¹ und R², die gleich oder verschieden sein können, für eine geradkettige oder verzweigte Alkanoylgruppe mit 2 bis 10 Kohlenstoffatomen oder eine Aralkanoylgruppe mit 7 bis 10 Kohlenstoffatomen steht, mit Phosphoroxychlorid oder Phosphoroxybromid in Gegenwart von N,N-Dimethylanilin, N-Ethylpiperidin oder Triethylamin und Triethylaminhydrochlorid, Tetrabutylammoniumchlorid, Tetraethylammoniumbromid, Tetramethylammoniumchlorid, Tetramethylammoniumbromid, Tetrabutylammoniumbromid oder Tetraethylammoniumchlorid umgesetzt und das Reaktionsprodukt hydrolysiert wird.

Die Erfindung wird im folgenden detailliert beschrieben, insbesondere in ihren bevorzugten Ausführungsformen. Unter der Bezeichnung Alkanoylgruppe mit 2 bis 10 Kohlenstoffatomen sind beispielsweise die Reste der folgenden Säuren zu verstehen:
Essigsäure, Propionsäure, n-Buttersäure, i-Buttersäure, n-Valeriansäure, i-Valeriansäure, Methylethylessigsäure, Trimethylessigsäure, Capronsäure, Caprylsäure, Caprinsäure, Heptansäure und Nonansäure. Mit der Bezeichnung Aralkanoylgruppe mit 7 bis 10 Kohlenstoffatomen sind z.B. die Reste folgender Verbindungen gemeint: Benzoesäure, Phenylessigsäure, Phenylpropansäure, Phenyl-n-buttersäure oder Phenyl-i-buttersäure.

Die erfindungsgemäße Herstellung der Verbindung der Formel I geht von 2,9-Diacylguanin (2-Acylamino-6-oxo-9-acyl-purin) aus. Dazu kann 2,9-Diacylguanin in einem polaren inerten Lösungsmittel, wie Acetonitril, Tetrahydrofuran, Dioxan, Nitromethan oder Dimethoxyethan suspendiert werden. Danach erfolgt die Zugabe der geeigneten Base, Triethylamin, N-Ethylpiperidin oder N,N-Dimethylanilin. Das molare Verhältnis von Base zu 2,9-Diacylguanin beträgt zweckmäßig 1 bis 6 zu 1, bevorzugt 1 bis 2 zu 1.

Anschließend wird ein Alkylhalogenidsalz zugefügt, ausgewählt aus der Gruppe Tetraethylammoniumchlorid, Triethylaminhydrochlorid, Tetraethylammoniumbromid, Tetramethylammoniumchlorid, Tetramethylammoniumbromid, Tetrabutylammoniumbromid und Tetrabutylammoniumchlorid. Das molare Verhältnis von Alkylhalogenidsalz zu 2,9-Diacylguanin beträgt 1 bis 3 zu 1, bevorzugt 1,5 bis 2 zu 1.

Dann wird das Halogenierungsmittel Phosphoroxychlorid oder Phosphoroxybromid zugegeben. Das molare Verhältnis von Halogenierungsmittel zu 2,9-Diacylguanin beträgt 1 bis 10 zu 1, bevorzugt 4 bis 6 zu 1. Die Zugabe des Halogenierungsmittels erfolgt in einem Temperaturbereich von -10°C bis 30°C, bevorzugt zwischen 0°C und 5°C. Nach Zugabe des Halogenierungsmittels wird die Temperatur des Reaktionsgemisches zwischen 10°C und 80°C, bevorzugt zwischen 15°C und 30°C, gehalten. Die Reaktionszeit beträgt 10 Minuten bis 72 Stunden, bevorzugt 6 bis 48 Stunden.

Die Reihenfolge der einzelnen Verfahrensschritte kann auch variiert werden. Einem Fachmann fällt es nicht schwer die optimale Reihenfolge für den Reaktionsprozeß zu finden.

Der Reaktionsverlauf wird durch Probeentnahme und quantitative Analyse des entstandenen 2-Acylamino-6-halogen-9-acyl-purins mit Hilfe der Hochdruckflüssigchromatographie (HPLC) verfolgt.

Die Hydrolyse der Acylgruppe an Position 9 des Purinringes erfolgt z.B. durch Einleiten des Reaktionsgemisches in Wasser. Dadurch wird das Reaktionsprodukt (2-Acylamino-6-halogen-9-acyl-purin) zur Verbindung der Formel I hydrolysiert. Die Temperatur wird zwischen -10°C und 30°C, bevorzugt zwischen 0°C und 5°C, gehalten. Gleichzeitig wird mit einer geeigneten Base der pH-Wert der Lösung zwischen 3 und 8, bevorzugt zwischen 4 und 7, eingestellt. Geeignete Basen sind z.B. Ammoniaklösung, Natriumhydroxid, Kaliumhydroxid, Natriumcarbonat, Kaliumcarbonat, Calciumhydroxid, Natriumhydrogencarbonat oder Kaliumhydrogencarbonat. Bevorzugt werden 12 %ige Ammoniaklösung, 2 N Natriumcarbonatlösung oder 2 N Natriumhydroxidlösung verwendet.

Nachdem sich ein konstanter pH-Wert eingestellt hat, wird der Reaktionsansatz noch weiter gerührt. Die Temperatur des Ansatzes wird zwischen -10°C und 25°C, bevorzugt zwischen -5°C und 5°C, gehalten. Es wird zwischen 0,5 und 24 Stunden, bevorzugt zwischen 1 bis 10 Stunden, gerührt. Dann kann die Verbindung der Formel I in hoher Reinheit durch Filtration aus dem Reaktionsansatz gewonnen werden.

Die Herstellung von 2,9-Diacetylguanin (2-Acetamino-6-oxo-9-acetyl-purin) aus Guanin durch Acetylierung wurde bereits beschrieben (H. Hrebabecky, J. Farkas, Nucleic Acid Chemistry, 1978, 13-14). Dabei wird Guanin in N-Methyl-2-pyrrolidon mit Essigsäureanhydrid acetyliert. Homologe 2,9-Diacylguanine können in analoger Weise gewonnen werden.

Im Vergleich zu den bisher bekannten Wegen zur Synthese von 2-Acylamino-6-halogen-purinen eröffnet das erfindungsgemäße Verfahren den Vorteil, das Endprodukt in hoher Reinheit und Ausbeute zu erhalten. Da das Endprodukt direkt durch Filtration aus der Reaktionslösung gewonnen werden kann, werden auch langwierige Extraktionsprozesse vermieden.

### Beispiel 1:

Zu einer Suspension aus 42,6 g (0,18 Mol) 2,9-Diacetylguanin in 450 ml Acetonitril werden 45,0 g (0,27 Mol) Tetraethylammoniumchlorid und 22,8 g (0,2 Mol) N-Ethylpiperidin hinzugefügt. Anschließend werden bei einer Temperatur von 0°C bis 5°C des Reaktionsansatzes 165,3 g (1,08 Mol) Phosphoroxychlorid zudosiert. Der Ansatz wird bei 25°C 20 Stunden gerührt, dann so in 1,25 l Wasser eingeleitet, daß 15°C nicht überschritten werden und gleichzeitig wird mit 12 %iger Ammoniaklösung der pH-Wert zwischen 4,8 und 5,5 gehalten. Bei einem konstanten pH-Wert von 5 wird bei 0°C bis 5°C 1 Stunde gerührt, danach das Produkt abfiltriert, mit Wasser gewaschen und im Vakuum bei 50°C getrocknet. Man erhält 34,1 g 2-Acetamino-6-chlor-purin. Die Bestimmung des Endproduktes erfolgt mit Hilfe der HPLC auf einer RP-18-Säule in einem Reaktionsgemisch von Wasser, Acetonitril und Essigsäure im Verhältnis 6:1:0,5.
- Ausbeute:: 87,3 % d.Th.
- Reinheit:: 97,2 % (HPLC)
- ¹H-NMR (in DMSO)::

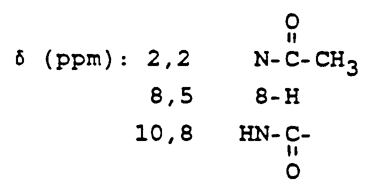

### Beispiel 2:

Zu einer Suspension aus 42,6 g (0,18 Mol) 2,9-Diacetylguanin in 450 ml Acetonitril werden 60,0 g (0,36 Mol) Tetraethylammoniumchlorid und 20,3 g (0,2 Mol) Triethylamin zugefügt. Danach werden bei einer Temperatur des Reaktionsansatzes von 0°C bis 5°C 165,3 g (1,08 Mol) Phosphoroxychlorid zugegeben. Der Ansatz wird bei 25°C 20 Stunden gerührt und anschließend wie im Beispiel 1 aufgearbeitet. Man erhält 30,9 g 2-Acetamino-6-chlor-purin.
- Ausbeute:: 80,6 % d.Th.
- Reinheit:: 99,1 % (HPLC)
- ¹H-NMR: (siehe Beispiel 1)

### Beispiel 3:

Zu einer Suspension aus 42,6 g (0,18 Mol) 2,9-Diacetylguanin in 450 ml Acetonitril werden 49,8 g (0,36 Mol) Triethylaminhydrochlorid und 20,3 g (0,2 Mol) Triethylamin zugegeben. Bei einer Temperatur von 0°C bis 5°C des Reaktionsansatzes werden 165,3 g (1,08 Mol) Phosphoroxychlorid zudosiert. Der Ansatz wird bei 25°C 42 Stunden gerührt und anschließend wie in Beispiel 1 aufgearbeitet. Man erhält 25,1 g 2-Acetamino-6-chlor-purin.
- Ausbeute:: 64,5 % d.Th.
- Reinheit:: 97,6 % (HPLC)
- ¹H-NMR: (siehe Beispiel 1)

## Patentansprüche

1. Verfahren zur Herstellung der Verbindung der Formel I, 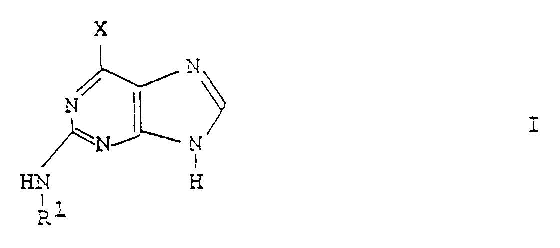 in der R¹ für eine geradkettige oder verzweigte Alkanoylgruppe mit 2 bis 10 Kohlenstoffatomen oder eine Aralkanoylgruppe mit 7 bis 10 Kohlenstoffatomen und X für Chlor oder Brom steht, dadurch gekennzeichnet, daß 2,9-Diacylguanin der Formel II, 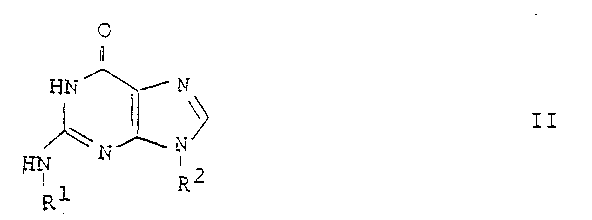 in der R¹ und R², die gleich oder verschieden sein können, für eine geradkettige oder verzweigte Alkanoylgruppe mit 2 bis 10 Kohlenstoffatomen oder eine Aralkanoylgruppe mit 7 bis 10 Kohlenstoffatomen steht, mit Phosphoroxychlorid oder Phosphoroxybromid in Gegenwart von N,N-Dimethylanilin, N-Ethylpiperidin oder Triethylamin und Triethylaminhydrochlorid, Tetrabutylammoniumchlorid, Tetraethylammoniumbromid, Tetramethylammoniumchlorid, Tetramethylammoniumbromid, Tetrabutylammoniumbromid oder Tetramethylammoniumchlorid umgesetzt und das Reaktionsprodukt hydrolysiert wird.

2. Verfahren nach Anspruch 1, dadurch gekennzeichnet, daß die Reaktion bei einer Temperatur zwischen -10°C und 80°C durchgeführt wird.

3. Verfahren nach einem oder mehreren der Ansprüche 1 bis 2, dadurch gekennzeichnet, daß das molare Verhältnis von Halogenierungsmittel zur Verbindung der Formel II 1 bis 10 zu 1 beträgt.

4. Verfahren nach Anspruch 3, dadurch gekennzeichnet, daß das molare Verhältnis 4 bis 6 zu 1 beträgt.

5. Verfahren nach einem oder mehreren der Ansprüche 1 bis 4, dadurch gekennzeichnet, daß das molare Verhältnis von Salz zur Verbindung der Formel II 1 bis 3 zu 1 beträgt.

6. Verfahren nach einem oder mehreren der Ansprüche 1 bis 5, dadurch gekennzeichnet, daß das molare Verhältnis von Base zur Verbindung der Formel II 1 bis 6 zu 1 beträgt.

7. Verfahren nach einem oder mehreren der Ansprüche 1 bis 6, dadurch gekennzeichnet, daß die Hydrolyse bei einem pH-Wert zwischen 3 und 8 erfolgt.

8. Verfahren nach Anspruch 7, dadurch gekennzeichnet, daß die Hydrolyse bei einer Temperatur zwischen -10°C und 30°C erfolgt.

## Claims

1. A process for the preparation of the compound of the formula I 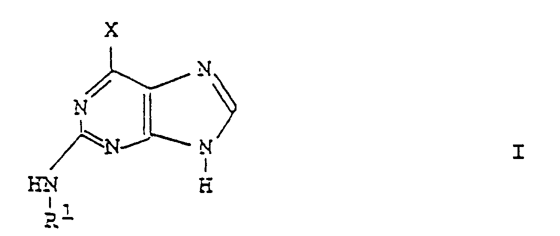 in which R¹ is a straight-chain or branched alkanoyl group having 2 to 10 carbon atoms or an aralkanoyl group having 7 to 10 carbon atoms and X is chlorine or bromine, which comprises reacting 2,9-diacylguanine of the formula II 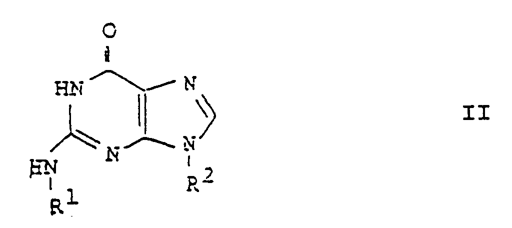 in which R¹ and R², which can be the same or different, are a straight-chain or branched alkanoyl group having 2 to 10 carbon atoms or an aralkanoyl group having 7 to 10 carbon atoms, with phosphorus oxychloride or phosphorus oxybromide in the presence of N,N-dimethylaniline, N-ethylpiperidine or triethylamine and triethylamine hydrochloride, tetrabutylammonium chloride, tetraethylammonium bromide, tetramethylammonium chloride, tetramethylammonium bromide, tetrabutylammonium bromide or tetraethylammonium chloride and hydrolyzing the reaction product.

2. The process as claimed in claim 1, wherein the reaction is carried out at a temperature between - 10°C and 80°C.

3. The process as claimed in one or more of claims 1 to 2, wherein the molar ratio of halogenating agent to the compound of the formula II is 1-10 to 1.

4. The process as claimed in claim 3, wherein the molar ratio is 4-6 to 1.

5. The process as claimed in one or more of claims 1 to 4, wherein the molar ratio of salt to the compound of the formula II is 1-3 to 1.

6. The process as claimed in one or more of claims 1 to 5, wherein the molar ratio of base to the compound of the formula II is 1-6 to 1.

7. The process as claimed in one or more of claims 1 to 6, wherein the hydrolysis is carried out at a pH between 3 and 8.

8. The process as claimed in claim 7, wherein the hydrolysis is carried out at a temperature between - 10°C and 30°C.

## Revendications

1. Procédé pour la préparation du composé de formule I, 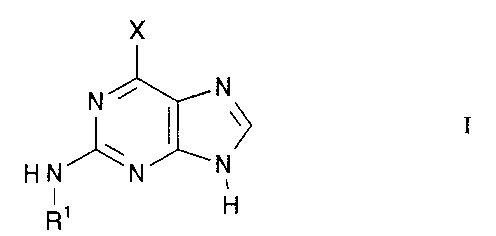 dans laquelle R¹ représente un groupe alcanoyle linéaire ou ramifié à 2 à 10 atomes de carbone ou un groupe aralcanoyle à 7 à 10 atomes de carbone et X représente un atome chlore ou de brome, caractérisé en ce que l'on fait réagir une 2,9-diacylguanine de formule II, 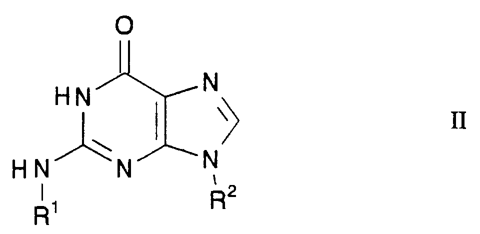 dans laquelle R¹ et R² qui peuvent être identiques ou différents, représentent un groupe alcanoyle linéaire ou ramifié à 2 à 10 atomes de carbone ou un groupe aralcanoyle à 7 à 10 atomes de carbone, avec du phosphoroxychlorure ou du phosphoroxybromure en présence de N,N-diméthylaniline, N-éthylpipéridine ou de triéthylamine, et de chlorhydrate de triéthylamine, de chlorure de tétrabutylammonium, de bromure de tétraéthylammonium, de chlorure de tétraméthylammonium, de bromure de tétraméthylammonium, de bromure de tétrabutylammonium ou de chlorure de tétraéthylammonium, et le produit de la réaction est hydrolysé.

2. Procédé selon la revendication 1, caractérisé en ce que la réaction est conduite à une température comprise entre -10°C et 80°C.

3. Procédé selon une ou plusieurs des revendications 1 à 2, caractérisé en ce que la proportion molaire de l'agent d'halogénation et du composé de formule II est de 1 à 10 pour 1.

4. Procédé selon la revendication 3, caractérisé en ce que la proportion molaire est de 4 à 6 pour 1.

5. Procédé selon une ou plusieurs des revendications 1 à 4, caractérisé en ce que la proportion molaire du sel et du composé de formule II est de 1 à 3 pour 1.

6. Procédé selon une ou plusieurs des revendications 1 à 5, caractérisé en ce que la proportion molaire de la base et du composé de formule II est de 1 à 6 pour 1.

7. Procédé selon une ou plusieurs des revendications 1 à 6, caractérisé en ce que l'hydrolyse a lieu à un pH compris entre 3 et 8.

8. Procédé selon la revendication 7, caractérisé en ce que l'hydrolyse a lieu à une température comprise entre -10°C et 30°C.
